# EUROPEAN PATENT APPLICATION

(11) **EP 3 505 128 A1**
(43) Date of publication of application: **03.07.2019**
(21) Application number: 17210687.4
(22) Date of filing: 27.12.2017
(51) Int. Cl.: A61B 18/20, A61B 18/00

(54) **CUTTING ASSEMBLIES FOR HAIR CUTTING DEVICES**

(71) Applicant: Koninklijke Philips N.V., 5656 AE Eindhoven (NL)
(72) Inventor: Jurna, Martin, 5656 AE Eindhoven (NL); Palero, Jonathan Alambra, 5656 AE Eindhoven (NL); Thumma, Kiran Kumar, 5656 AE Eindhoven (NL); Boamfa, Marius Iosif, 5656 AE Eindhoven (NL); Johnson, Mark Thomas, 5656 AE Eindhoven (NL)
(74) Representative: de Haan, Poul Erik

(57) **Abstract**

The invention discloses a cutting assembly for use in a hair cutting device (100), the cutting assembly comprising an optical waveguide (102) having a sidewall, wherein a portion of the sidewall forms a cutting face for contacting a hair; and a movement mechanism (108) for effecting movement of the optical waveguide relative to the hair, in a direction substantially parallel to an optical axis of the optical waveguide (102). The invention also discloses a cutting assembly for use in a hair cutting device, the cutting assembly comprising an optical waveguide (102) having a sidewall, wherein a portion of the sidewall forms a cutting face for contacting a hair; a hair support for supporting the hair relative to the optical waveguide; and a movement mechanism (108) for effecting movement of the hair support relative to the optical waveguide (102), in a direction substantially parallel to an optical axis of the optical waveguide. A hair cutting device (100) is also disclosed.

## Description

### FIELD OF THE INVENTION

The invention relates to a cutting assembly for use with a hair cutting device for cutting (e.g. shaving) hair on the body of the subject and, in particular, to a cutting assembly having a mechanism for causing relative movement. The invention also relates to a hair cutting device comprising such a cutting assembly.

### BACKGROUND OF THE INVENTION

Shaving devices for cutting or shaving hair on a body of a subject typically make use of one or more blades that cut hairs as the blade is moved across the skin of the subject. The blades can be static within the device, for example as in a wet razor, whereas in other types of devices, for example electric shavers, one or more blade elements can be actuated (e.g. rotated or oscillated) in order to produce a cutting action.

However, an alternative type of shaving device has been proposed in WO 2014/143670 that makes use of laser light. In particular, a laser light source is provided that is configured to generate laser light having a wavelength selected to target a predetermined chromophore to effectively cut a hair shaft. A fiber optic is located on a shaving portion of the device that is positioned to receive the laser light from the laser light source at a proximal end, conduct the laser light from the proximal end toward a distal end, and emit the light out of a cutting region of the fiber optic and toward hair when the cutting region is brought in contact with the hair.

When light from the fiber optic couples into a hair to be cut, the temperature of the hair may rapidly increase. Consequently, the portion of the fiber optic that is in contact with the hair may also heat up. If the increase in temperature of the fiber optic is too great, or is too rapid, then the fiber optic may become damaged.

Therefore, it is desirable to have a cutting device having an optical waveguide cutting arrangement, which has a reduced likelihood of overheating, particularly in a localized area of the optical waveguide.

### SUMMARY OF THE INVENTION

In order to cut or melt hair, sufficient optical energy needs to be delivered through a cutting element of the cutting device. However, to reduce the risk of localized overheating in a particular portion of the optical waveguide during the cutting process, it is desirable to prevent prolonged contact between a particular portion of the optical waveguide and the hair being cut. According to embodiments in the present disclosure, this is achieved by introducing relative movement between the cutting element of the device and the hair or hairs to be cut. Specifically, relative movement is introduced in a direction substantially parallel to the optical axis of the optical waveguide. In this way, heat generated during the cutting process is distributed over a length of the optical waveguide rather than being concentrated in a particular area.

According to a first aspect, a cutting assembly for use in a hair cutting device comprises an optical waveguide having a sidewall, wherein a portion of the sidewall forms a cutting face for contacting a hair. The cutting device also comprises a movement mechanism for effecting movement of the optical waveguide relative to the hair, in a direction substantially parallel to an optical axis of the optical waveguide.

In some embodiments, the movement mechanism may be configured to move the optical waveguide between a first position and a second position in a direction substantially parallel to the optical axis of the optical waveguide.

The cutting assembly may further comprise a hair support for supporting the hair relative to the optical waveguide.

According to a second aspect, a cutting assembly for use in a hair cutting device comprises an optical waveguide having a sidewall, wherein a portion of the sidewall forms a cutting face for contacting a hair. The cutting device also comprises a hair support for supporting the hair relative to the optical waveguide; and a movement mechanism for effecting movement of the hair support relative to the optical waveguide, in a direction substantially parallel to an optical axis of the optical waveguide.

The movement mechanism may be configured to induce a controlled vibration in the hair support.

In some embodiments, the movement mechanism may be configured to move the hair support between a first position and a second position in a direction substantially parallel to the optical axis of the optical waveguide.

The movement mechanism may, in some embodiments, be configured to effect movement at a defined speed or at a speed exceeding the defined speed. In some embodiments, the movement mechanism may be configured to effect movement at a speed exceeding a speed of movement of the cutting assembly over a surface containing the hair intended to be cut.

The movement mechanism may comprise at least one of: a piezoelectric mechanism, a loudspeaker arrangement, a linear motor, and a capacitive micro-machined ultrasonic transducer, CMUT, device.

In some embodiments, the movement effected by the movement mechanism may comprise a controlled vibration.

In some embodiments, the movement mechanism may be configured to effect a controlled vibration having a vibrational frequency of between around 50 Hz and 15 kHz.

The optical waveguide may comprise an optical fiber.

According to a third aspect, a hair cutting device for cutting hair on a body of a subject comprises a light source for generating laser light at one or more specific wavelengths corresponding to wavelengths absorbed by one or more chromophores in hair; and a cutting assembly having a cutting element coupled to the light source to receive laser light, the cutting assembly comprising a cutting assembly as described herein.

In some embodiments, the hair cutting device may further comprise a sensor for measuring the speed of movement of the hair cutting device over the body of the subject.

The hair cutting device may, in some embodiments, further comprise a processor configured to vary the speed of movement effected by the movement mechanism based on the measured speed of movement of the hair cutting device.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified schematic of a hair cutting device according to embodiments;
Fig. 2 is a pair of schematic drawings showing different views of an exemplary hair cutting device according to embodiments;
Fig. 3 is a graph illustrating the refractive index of hair;
Fig. 4 is a simplified schematic of an example of a cutting assembly according to embodiments;
Fig. 5 is an illustration showing heat distribution profiles for various optical waveguides;
Fig. 6 is a simplified schematic of a further example of a cutting assembly according to embodiments;
Fig. 7 is a simplified schematic of a further example of a cutting assembly according to embodiments; and
Fig. 8 is a simplified schematic of an example of a hair cutting device according to embodiments.

### DETAILED DESCRIPTION OF THE EMBODIMENTS

As noted above, the present invention provides an improvement in the cutting ability and efficiency of a laser light-based shaving device, for example as described in WO 2014/143670. In particular, it has been recognized that by reducing the amount of time that a particular portion of a cutting element is in contact with a hair to be cut, the increase in temperature of that portion of the cutting element can be reduced, thereby reducing the chance of failure of the cutting element resulting from damage caused by too much heat.

It will be appreciated that the invention is applicable to shaving devices (e.g. razors or electric shavers), and any other type of device that is used to cut hair (e.g. hair clippers), even if those devices do not necessary aim to provide a 'clean shave' (i.e. to remove hair at the level of the skin).

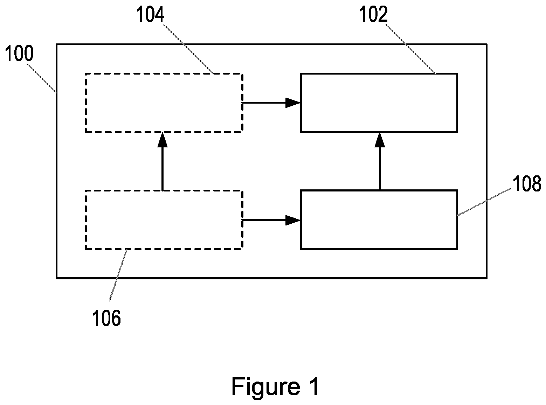

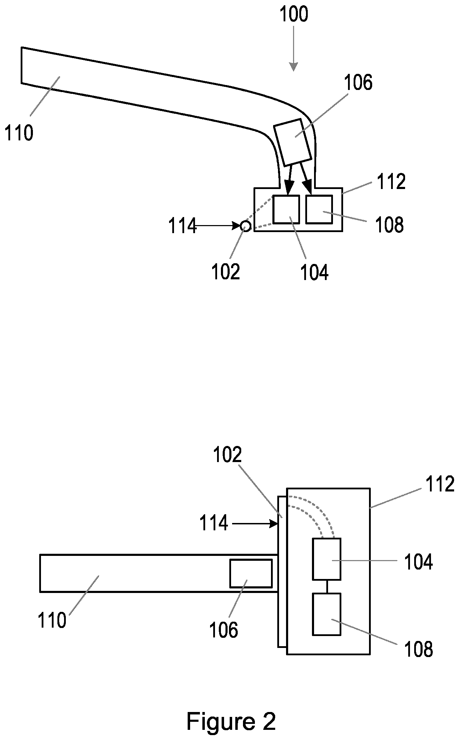

Fig. 1 is a block diagram of a hair cutting device 100 according to an embodiment of the invention. Fig. 2 shows a hair cutting device 100 in the form of a handheld razor according to an exemplary embodiment of the invention. The hair cutting device 100 is for cutting (e.g. shaving) hair on a body of a subject. The subject may be a person or an animal. The hair may be facial hair (i.e. hair on the subject's face), or hair on the subject's head or other part of their body (legs, chest, etc.).

The hair cutting device 100 comprises a cutting element 102 that enables hair to be cut as the hair cutting device 100 is moved over the skin of a subject. The cutting element 102 is an optical waveguide 102 that is arranged on the hair cutting device 100 so that the optical axis of the optical waveguide 102 (i.e. the line along which light typically propagates through the optical waveguide 102) is generally perpendicular to the direction in which the hair cutting device 100 is moved so that hairs contact the sidewall of the optical waveguide 102 (the sidewall corresponding to at least a portion of the long edge of the optical waveguide 102) as the hair cutting device 100 is moved across the skin of the subject. In some embodiments, the optical waveguide 102 is an optical fiber, although those skilled in the art will be aware of other types of optical waveguide that can be used according to the invention, such as a slab waveguide, a strip waveguide or a photonic crystal waveguide. An optical fiber comprises a core, and in some embodiments also comprises a cladding, which may or may not fully encompass the core (e.g. part of the core may be exposed).

A light source 104 is provided in the hair cutting device 100 that generates laser light at one or more specific wavelengths. The light source 104 is optically coupled to the optical waveguide 102. The laser light generated by the light source 104 is coupled into the optical waveguide 102 (and specifically coupled into an end of the optical waveguide so that the laser light propagates through the optical waveguide).

The light source 104 is configured to generate laser light at one or more specific wavelengths that can be used to cut or burn through hair. In particular, each wavelength corresponds to the wavelength of light absorbed by a chromophore that is found in hair. As is known, a chromophore is the part of a molecule that provides the molecule with its color. Thus, the laser light will be absorbed by the chromophore and converted into heat which will melt or burn the hair or otherwise destroy the bonds in the molecules of the hair, and it is this melting or burning that provides the cutting action of the hair cutting device 100.

Suitable chromophores that can be targeted by the laser light generated by the light source 104 include, but are not limited to, melanin, keratin and water. Suitable wavelengths of laser light that can be used include, but are not limited to, wavelengths selected from the range 380 nm (nanometers) to 500 nm and 2500 nm to 3500 nm. Those skilled in the art will be aware of the wavelengths of light that are absorbed by these chromophores, and thus also the specific wavelengths of light that the light source 104 should generate for this purpose, and further details are not provided herein.

In some embodiments the light source 104 can be configured to generate laser light at a plurality of wavelengths (either simultaneously or sequentially), with each wavelength being selected to target a different type of chromophore. This can improve the cutting action of the optical waveguide 102 since multiple types of molecules in the hair may be burnt using the laser light. Alternatively multiple light sources 104 can be provided that each generate laser light at a respective wavelength, and each light source 104 can be coupled to a respective optical waveguide 102 to provide multiple cutting elements 102 in the device 100.

The hair cutting device 100 may also comprise a control unit 106 to control the operation of the hair cutting device 100. The control unit 106 may be connected to the light source 104 to control the activation and deactivation of the light source 104 (and in some embodiments control the wavelength and/or intensity of the light generated by the light source 104). The control unit 106 may activate and deactivate the light source 104 in response to an input from a user of the hair cutting device 100. The control unit 106 can comprise one or more processors, processing units, multi-core processors or modules that are configured or programmed to control the hair cutting device 100. In some embodiments, the control unit may receive instructions from other controllers or processors remote from the device 100.

The hair cutting device 100 also includes a movement mechanism 108 configured to move the optical waveguide 102 relative to the hair cutting device. Specifically, the movement mechanism 108 is for effecting movement either of the optical waveguide relative to a hair to be cut, or of a hair support arranged to support the hair, relative to the optical waveguide, as is discussed in detail below. The movement mechanism 108 may be controlled by the control unit 106. In some embodiments, the hair cutting device 100 may also include a sensor (not shown in Figs. 1 or 2) for measuring the speed of movement of the device 100 over a surface, as is discussed in greater detail below. The optical waveguide 102 and the movement mechanism 108 may be considered to form components of a cutting assembly which, in some embodiments, may be detachable from the hair cutting device 100, and may be enclosed in a separate unit or housing. In some embodiments, the hair support may also form part of the cutting assembly.

As noted above, Fig. 2 shows a hair cutting device 100 that is in the form of a handheld wet razor. Fig. 2 shows a side view and a bottom view of the razor 100. The razor 100 comprises a handle 110 for the subject (or other user of the device 100) to hold, and a head portion 112 that includes the cutting element 102 (optical waveguide/fiber) and the movement mechanism 108. As shown, the optical waveguide 102 is arranged along an edge of the head portion, and a part of the optical waveguide 102 forms (or corresponds to) a cutting face 114. The cutting face 114 is the part of the optical waveguide 102 that is intended to come into contact with hair as the hair cutting device 100 is moved across the skin of the subject. The light source 104 and the control unit 106 are shown as being incorporated into the head portion 112 and handle 110 respectively, but it will be appreciated that the positions of these components or the movement mechanism 108 in the hair cutting device 100 as shown in Fig. 2 is not limiting. Likewise it will be appreciated that the embodiment shown in Fig. 2 is merely an example, and the invention can be incorporated or used in any type of hair cutting device 100 that comprises an optical waveguide cutting element 102 as described herein. As noted above, the optical waveguide 102 and the movement mechanism 108 may form part of a cutting assembly which may itself form a part of, or be detachably connected to, the head portion 112 and/or to the handle 110.

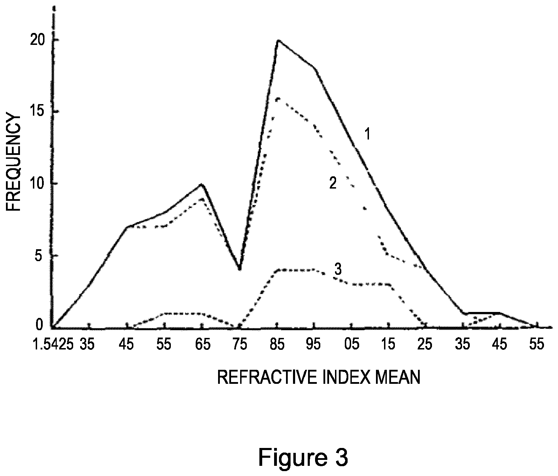

The graph in Fig. 3 illustrates the refractive index of hair, which can be found in a paper by M. D. Greenwell, A. Willner, Paul L. Kirk: Human Hair Studies: III. Refractive Index of Crown Hair, 31 Am. Inst. Crim. L. & Criminology 746 (1940-1941). Curve 1 is a composite line, curve 2 is a line representing the refractive index for Caucasian people, and curve 3 is a line representing the refractive index for non-Caucasian people. Thus, it can be seen that the refractive index of hair is between (approximately) 1.545 and 1.555, although there will be variation between individuals. For example the above paper also recognizes that the refractive index of hair can depend on the sex of the subject, e.g. the refractive index of hair on a female is generally higher than the refractive index of hair on a male.

As is known, the optical waveguide 102 acts as a waveguide for the light coupled from the light source 104 through the occurrence of total internal reflection, since the refractive index of air is lower than that of the optical waveguide 102. However, if an object that has a refractive index higher than the optical waveguide 102 is put into contact with the optical waveguide 102, then the total internal reflection is 'frustrated' and light can couple from the optical waveguide 102 into that object. Thus, in order for light to be coupled into a hair from the optical waveguide 102 (to provide the cutting action according to the invention), the optical waveguide 102 should generally have the same or a lower refractive index than hair at the point at which the hair contacts the optical waveguide 102. Thus, the optical waveguide 102 should have the same or a lower refractive index than hair at least at the cutting face 114 portion of the optical waveguide 102. Preferably the refractive index of the optical waveguide 102 at the cutting face 114 is the same as that of hair since that provides the best coupling of light from the optical waveguide 102 to the hair. However, in cases where the refractive index of the optical waveguide 102 is higher than an object (e.g. a hair) in contact with the optical waveguide, less efficient output coupling can still be achieved by increasing the numerical aperture (NA) and, preferably, using the full NA. One technique which may be used to reach the full NA is to introduce local tapering in the optical waveguide at or near to the cutting region.

Thus, in some embodiments, the refractive index of the optical waveguide 102 at least at the cutting face 114 is equal to or lower than 1.56. More preferably the refractive index of the optical waveguide 102 at least at the cutting face 114 is equal to or lower than 1.55. Even more preferably, the refractive index of the optical waveguide 102 at least at the cutting face 114 is equal to or lower than 1.54, since this refractive index is below the refractive indices identified in Fig. 3.

In some embodiments, a lower bound for the refractive index of the optical waveguide 102 at the cutting face 114 can be 1.48, 1.51, 1.53 or 1.54.

A range of values from which the refractive index of the optical waveguide 102 is selected can be formed from any combination of the upper and lower refractive index bounds set out in the preceding paragraphs.

The optical waveguide/fiber 102 can be made from any suitable material or combination of materials. For example optical waveguides/fibers can be composed of or comprise silica, fluoride glass, phosphate glass, chalcogenide glass, crown glass (such as BK7) and/or crystals (such as sapphire or yttrium aluminum garnet (YAG)).

As noted above, while the optical waveguide/fiber 102 is in contact with a hair to be cut, the optical waveguide is caused to heat up significantly and, if the increase in temperature is too great, the optical waveguide may be damaged. The reason for the increase in temperature of the waveguide is that, as melting of the hair is initiated, the area of contact between the hair and the optical waveguide is increased and, as a result, more light from the waveguide is able to couple into the hair, causing a further increase in temperature. Therefore, it has been recognized that preventing prolonged periods of contact between a particular portion of the optical waveguide and a strand of hair to be cut can prevent significant temperature increases in the optical waveguide and, therefore, can lower the likelihood that the optical waveguide will be damaged by high temperatures. Accordingly, embodiments in the present disclosure involve causing relative movement (e.g. vibrational movement) between the optical waveguide and the hair to be cut. Specifically, the relative movement between the optical waveguide and the hair to be cut is in a direction either substantially parallel to an optical axis of the optical waveguide or substantially perpendicular to an intended direction of movement of the hair cutting device over the body of the subject.

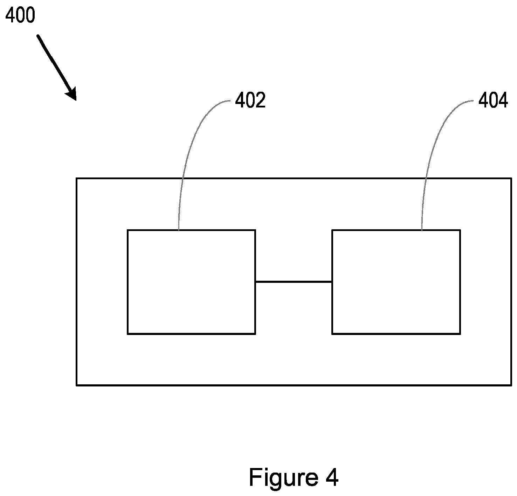

According to a first aspect, embodiments disclosed herein relate to a cutting assembly. Figure 4 is a simplified schematic of such a cutting assembly 400. The cutting assembly 400 is for use in a hair cutting device, such as the hair cutting device 100 discussed above. The cutting assembly 400 comprises an optical waveguide 402 having a side wall, wherein a portion of the sidewall forms a cutting face for contacting a hair. The optical waveguide 402 may comprise, or be similar to, the optical waveguide 102 discussed herein. The cutting assembly 400 further comprises a movement mechanism 404 for effecting the movement of the optical waveguide 402 relative to the hair, in a direction substantially parallel to an optical axis of the optical waveguide. The movement mechanism 404 may comprise, or be similar to, the movement mechanism 108 discussed herein.

By moving the optical waveguide 402 along its optical axis (e.g. in a cyclic or repetitive motion between a first position in the second position) as it engages a hair, the portion of the optical waveguide 402 that contacts the hair being cut varies. In other words, the hair being cut does not remain in contact with the same portion of the optical waveguide 402 during the cutting duration. In this way, as the hair to be cut heats up during the cutting process, and transfers its heat to the optical waveguide 402, the heat is transferred over a length of the optical waveguide, rather than being focused at one particular portion of the optical waveguide. Thus, it is distributed more widely throughout the optical waveguide 402, reducing the chance that the temperature of one particular portion of the optical waveguide, increases beyond the glass transition temperature (Tg), at which temperature the optical waveguide is more liable to break. While the optical waveguide could alternatively be moved in a direction substantially perpendicular to its optical axis in order to achieve a similar effect, by moving the optical axis in a direction substantially parallel to its optical axis, the chance of damaging the optical waveguide as it moves is reduced.

In some embodiments, the movement mechanism 404 may be configured to move the optical waveguide between the first position and a second position in a direction substantially parallel to the optical axis of the optical waveguide 402. Thus, the optical waveguide 402 may move back and forth between the first and second positions. In some embodiments, the movement mechanism 404 may be configured to induce a controlled vibration in the optical waveguide 402.

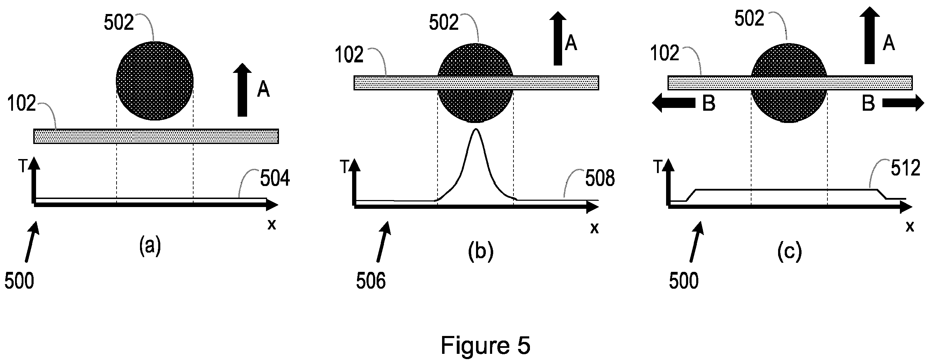

The benefits of effecting relative movement between the optical waveguide 402 and the hair to be cut can be seen from the examples shown in Fig. 5. Fig. 5a includes a graph 500 showing the distribution of temperature, T, over the length of the optical waveguide 102, in a direction x, as the optical waveguide moves towards a hair 502. The arrow A denotes the direction of motion of the optical waveguide towards the hair 502. In this example, the optical waveguide 102 is not in contact with the hair 502, so no light energy is coupling out of the optical waveguide into the hair. Accordingly, the temperature of the optical waveguide is approximately constant and relatively low over its length, as represented by the curve 504 in the graph 500.

In the example represented in Fig. 5b, the cutting assembly has been moved towards the hair to be cut, bringing the optical waveguide 102 into contact with the hair 502. The temperature distribution profile or this example is shown in a graph 506. In this example, since there is no relative lateral movement (i.e. along the optical axis of the optical waveguide 102) between the optical waveguide and the hair to be cut, the temperature of the optical waveguide increases at the portion that is in contact with the hair 502. This temperature increase is represented by a peak in a curve 508 of the graph 506. As noted above, if the increase in temperature of a particular portion of the optical waveguide 102 is too rapid, then a defect may form in the optical waveguide, or the optical waveguide may become brittle and prone to damage. Similarly, if the temperature of a particular portion of the optical waveguide 102 increases beyond a particular temperature, such as the glass transition temperature, Tg, then the optical waveguide may become damaged.

Fig. 5c shows the effect of introducing relative movement between the optical waveguide 102 and hair 502 being cut. In this example, the optical waveguide 102 is being moved (e.g. vibrated) backwards and forwards along the optical axis of the optical waveguide, in the directions denoted by the arrows B. Consequently, as the optical waveguide 102 is moved towards the hair 502, in the direction A, more of the optical waveguide contacts the hair, as compared to the example shown in Fig. 5b, and any particular portion of the optical waveguide remains in contact with the hair for a relatively smaller amount of time. As a result, the temperature of the optical waveguide prevented from increasing too rapidly, and is prevented from rising to a temperature which may result in damage. This is represented in a graph 510 by a temperature distribution curve 512, which shows that the temperature is distributed over a portion of the optical waveguide 102.

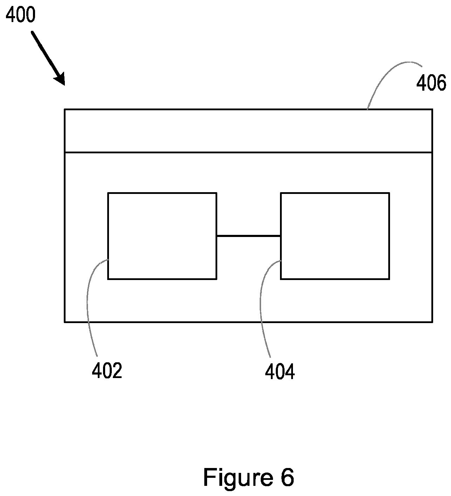

According to the embodiment shown in Fig. 4, the optical waveguide 102, 402 is caused, by the movement mechanism 404, to move (e.g. vibrate) backwards and forwards in a direction substantially parallel to its optical axis as the cutting assembly 400 is moved towards the hair to be cut (e.g. in a direction substantially perpendicular to the optical axis of the optical waveguide). In some embodiments, the cutting assembly and/or the hair cutting device in which the cutting assembly is incorporated may include a mechanism for holding the hair to be cut in a stationary position relative to the optical waveguide 402. Fig. 6 is a simplified schematic of an example of the cutting assembly 400 including such a mechanism. In the example shown in Fig. 6, the cutting assembly 400 further comprises a hair support 406 for supporting the hair relative to the optical waveguide 402. The hair support 406 may take various different forms. In general, however, the hair support 406 is configured to prevent movement of the hair to be cut while the optical waveguide 402 is in contact with the hair. In some examples, as the optical waveguide 402 is moved or vibrated along its optical axis, the hair may also be caused to move due to frictional forces between the optical waveguide and the hair. The hair support 406 may prevent the hair from moving with the optical waveguide 402 and, consequently, may help to prevent localized temperature increases within the optical waveguide.

In some embodiments, the hair support 406 may comprise a comb-like structure, having a plurality of teeth. A hair to be cut may enter the hair support 406 between a pair of adjacent teeth, and may be prevented from moving laterally (i.e. from side to side in the direction of the optical axis of the optical waveguide 402). In other embodiments, the hair support 406 may comprise a mesh-like material. Hairs may become caught or trapped in apertures in the mesh, thereby preventing movement of the hairs in a direction parallel to the optical axis of the optical waveguide 402. In other embodiments, other types of hair support 406 may be used to hold the hairs substantially stationary while the optical waveguide 402 is moved along its optical axis during the cutting process.

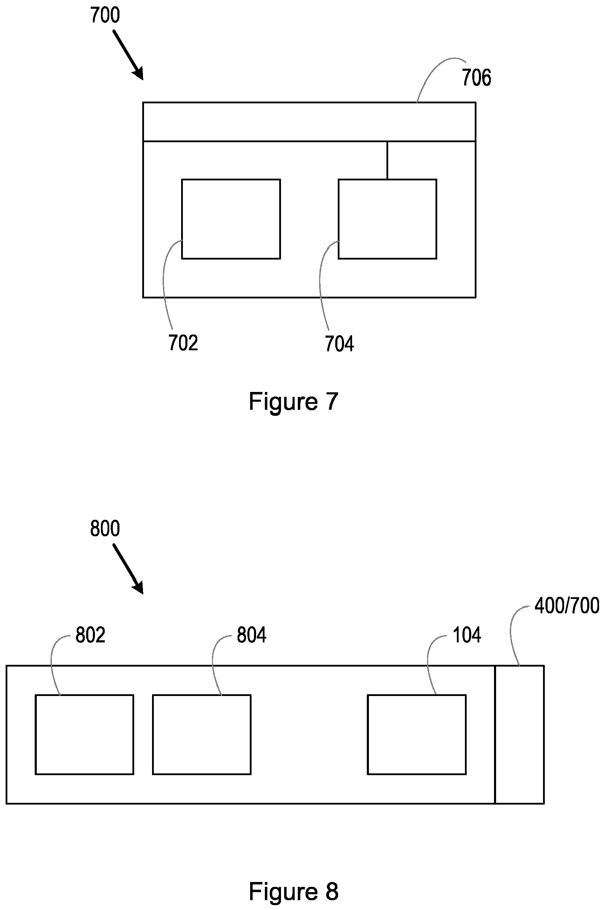

In embodiments described above, the optical waveguide 102, 402 may be moved relative to the hair and, in some embodiments, the hair may be held stationary to improve the cutting process. However, as noted above, an equivalent effect may be achieved by causing the hair to move, rather than the optical waveguide. Fig. 7 is a simplified schematic of an example of a cutting assembly 700 which has a different arrangement to the cutting assembly 400 discussed above, but which achieves the same effect. The cutting assembly 700 is for use in a hair cutting device, and comprises an optical waveguide 702 having a sidewall, wherein a portion of the sidewall forms a cutting face for contacting a hair; a hair support 706 for supporting the hair relative to the optical waveguide; and a movement mechanism 704 for effecting movement of the hair support relative to the optical waveguide, in a direction substantially parallel to an optical axis of the optical waveguide. Thus, in this example, rather than the optical waveguide 102, 402, 702 being caused to move, the hair support 706 is caused to move. In this way, a hair that is held, captured or trapped in the hair support 706 is caused to move along with the hair support, backwards and forwards in a direction substantially parallel to the optical axis of the optical waveguide 702. Since the optical waveguide 702 is not moved in a direction along its optical axis during cutting, any heat transfer from the hair to the optical waveguide 702 during cutting is spread over a length of the optical waveguide, thereby reducing the risk of a significant temperature increase in a particular localized portion of the optical waveguide.

By moving the hair support 706 (and therefore the hair) rather than the optical waveguide 702, there is less chance that the optical waveguide will become damaged as a result of rapid movements.

The movement mechanism 704 may be configured to move the hair support 706 between a first position and a second position in a direction substantially parallel to the optical axis of the optical waveguide 702. In some embodiments, the movement mechanism 704 may be configured to induce a controlled vibration in the hair support 706.

Whether the movement mechanism 404, 704 is configured to induce movement in the optical waveguide 402, 702 or the hair support 406, 706, it may function in a similar manner. The movement mechanism 404, 704 may be configured to move the optical waveguide 402, 702 or the hair support 406, 706 in various ways and at different speeds, depending on the intended use of the hair cutting device 100. As noted above, in some embodiments, the movement mechanism 404, 704 may comprise a vibration mechanism for inducing a controlled vibration. The optical waveguide 402, 702 or the hair support 406, 706 may be configured to vibrate at a defined speed, or vibrational frequency, and the distance that the waveguide or hair support moves during the vibration (i.e. the distance between the first position and the second position, or its amplitude) may be defined and controlled, for example, by the control unit 106. The movement mechanism 404, 704 may comprise any suitable mechanism for effecting controlled movement of the optical waveguide 402, 702 or the hair support 406, 706 in a direction substantially along the optical axis of the optical waveguide. In some embodiments, the movement mechanism 404, 704 may comprise at least one of: a piezoelectric mechanism, a loudspeaker arrangement, a linear motor, and a capacitive micro-machined ultrasonic transducer (CMUT) device.

As will be appreciated, if the movement mechanism 404, 704 comprises a vibration mechanism, then the movement of the optical waveguide 402, 702 or the hair support 406, 706 is likely to be relatively fast. In some embodiments, the movement mechanism 404, 704 may be configured (for example under control of the control unit 106) to effect movement (e.g. in the optical waveguide 402, 702 or the hair support 406, 706) at a defined speed or at a speed exceeding the defined speed. For example, in some embodiments, the movement mechanism 404, 704 may be configured to effect movement at a speed exceeding a speed of movement of the cutting assembly 400, 700 (or the cutting device) over a surface containing hair intended to be cut. The defined speed may be selected based on an average, or typical, speed of movement of a hair cutting device 100 over the skin of a subject during a shaving or hair cutting activity. For example, a typical shaving speed (i.e. speed of movement of the hair cutting device 100 by a user over the skin) for facial shaving might be 10 centimeters per second (cm/s) or 20 cm/s, whereas a typical shaving speed for shaving other body parts, such as legs, might be 50 cm/s. Thus, the movement mechanism 404, 704 may be configured to induce movement at a speed exceeding 10 cm/s, 20 cm/s or 50 cm/s, for example, depending on the intended use of the device 100.

In some embodiments, the speed of movement induced by the movement mechanism 404, 704 may be dynamic, such that the speed may be varied based on the speed of movement of the hair cutting device 100 over the skin. In such embodiments, the device 100 may include a speed sensor (see Fig. 8) for determining, in real time, the speed at which the device is moving over the skin of a subject. The speed sensor may, in some embodiments, include an optical sensor, such as a laser sensor for determining the rate at which the device 100 is moved over the skin. In some embodiments, a sensor similar to that used in an optical computer mouse may be used. The movement mechanism 404, 704 may be configured to move (or vibrate) the optical waveguide 402, 702 or the hair support 406, 706 in a cyclic manner at a speed based on the speed determined by the speed sensor. For example, the movement mechanism 404, 704 may be configured to induce movement (e.g. a vibration) at a speed or frequency equal to or exceeding the speed determined by the speed sensor.

It is estimated that, using a light source 104 having an output power of 1 Watt, the total time needed to fully cut a hair having a diameter of approximately 300 micrometers (in other words, the cutting time) using a cutting element 102, 402, 702 as described herein is between around 0.6 milliseconds (ms) to 3 ms. If a lower-powered light source is used, then the required cutting time may be longer, and if a higher-powered light source is used, then the required cutting time may be shorter. For the duration that the optical waveguide 402, 702 is in contact with the hair being cut, the optical waveguide or the hair is moved from side to side (i.e. backwards and forwards in a direction substantially parallel to the optical axis of the optical waveguide) so that the same portion of the optical waveguide does not remain in contact with the hair. This prevents a localized significant increase of temperature in the waveguide 402, 702. During the cutting time, the optical waveguide 402, 702 or the hair may, in some embodiments, be moved (e.g. displaced) by a distance of at least half the diameter of the hair to be cut. In order to achieve the desired cutting effectiveness and the intended relative movement between the hair and the optical waveguide 402, 702, the optical waveguide or the hair (e.g. via the hair support 406, 706) may be moved (e.g. vibrated) at a frequency of between approximately 50 Hz and approximately 15,000 Hz. In other words, the movement mechanism 404, 704 may be configured to effect a controlled vibration having a vibrational frequency of between around 50 Hz and 15 kHz. The vibrational frequency of the movement mechanism 404, 704 may be selected based on the intended use of the device 100. In some examples, the frequency of movement/vibration may be between approximately 500 Hz and 5000 Hz. In other examples, the frequency of movement/vibration may be between approximately 500 Hz and 2000 Hz.

As will be apparent from the embodiments disclosed herein, the optical waveguide may, in some embodiments, comprise an optical fiber.

In general, embodiments disclosed herein relate to a cutting assembly for use in a hair cutting device, the cutting assembly comprising an optical waveguide having a sidewall, wherein a portion of the sidewall forms a cutting face for contacting a hair. The cutting assembly also comprises a movement mechanism for effecting relative movement of at least one of the optical waveguide and the hair, in a direction substantially parallel to an optical axis of the optical waveguide.

In other words, the cutting assembly may comprise a movement mechanism arranged to create a relative movement of the optical waveguide and the hair with respect to each other. The movement mechanism may be arranged to effect movement of the optical waveguide in a direction substantially parallel to an optical axis of the optical waveguide; or the cutting assembly may further comprise a hair support for supporting the hair relative to the optical waveguide, and the movement mechanism may be arranged to effect movement of the hair support relative to the optical waveguide, in a direction substantially parallel to an optical axis of the optical waveguide.

According to another aspect, embodiments disclosed herein relate to a hair cutting device. Fig. 8 is a simplified schematic of a hair cutting device 800 for cutting hair on a body of a subject. The hair cutting device 800 comprises a light source 104 for generating laser light at one or more specific wavelengths corresponding to wavelengths absorbed by one or more chromophores in hair; and a cutting assembly 400, 700 having a cutting element 402, 702 coupled to the light source 104 to receive laser light, the cutting assembly comprising a cutting assembly as disclosed herein.

In some embodiments, the hair cutting device 800 may comprise a sensor 802 for measuring the speed of movement of the hair cutting device 800 over the body of the subject. As discussed above, the speed of movement determined by the sensor 802 may be used to set a speed of movement (e.g. vibration) caused by the movement mechanism 404, 704.

The hair cutting device 800 may, in some embodiments, further comprise a processor 804 configured to vary the speed of movement effected by the movement mechanism 404, 704 based on the measured speed of movement of the hair cutting device. In some embodiments, the processor 804 may be configured to perform other functions. For example, the processor 804 may comprise the control unit 106.

The processor 804 can comprise one or more processors, processing units, multi-core processors or modules that are configured or programmed to control the various components in the manner described herein.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A cutting assembly (400) for use in a hair cutting device, the cutting assembly comprising:
an optical waveguide (402) having a sidewall, wherein a portion of the sidewall forms a cutting face for contacting a hair; and
a movement mechanism (404) for effecting movement of the optical waveguide relative to the hair, in a direction substantially parallel to an optical axis of the optical waveguide.

2. A cutting assembly (400) according to claim 1, wherein the movement mechanism (404) is configured to move the optical waveguide (402) between a first position and a second position in a direction substantially parallel to the optical axis of the optical waveguide.

3. A cutting assembly (400) according to claim 2 or claim 3, further comprising a hair support (406) for supporting the hair relative to the optical waveguide.

4. A cutting assembly (700) for use in a hair cutting device, the cutting assembly comprising:
an optical waveguide (702) having a sidewall, wherein a portion of the sidewall forms a cutting face for contacting a hair;
a hair support (706) for supporting the hair relative to the optical waveguide; and
a movement mechanism (704) for effecting movement of the hair support relative to the optical waveguide, in a direction substantially parallel to an optical axis of the optical waveguide.

5. A cutting assembly (700) according to claim 4, wherein the movement mechanism (704) is configured to induce a controlled vibration in the hair support (706).

6. A cutting assembly (700) according to claim 4 or claim 5, wherein the movement mechanism (704) is configured to move the hair support (706) between a first position and a second position in a direction substantially parallel to the optical axis of the optical waveguide.

7. A cutting assembly (400, 700) according to any of the preceding claims, wherein the movement mechanism (404, 704) is configured to effect movement at a defined speed or at a speed exceeding the defined speed.

8. A cutting assembly (400, 700) according to any of the preceding claims, wherein the movement mechanism (404, 704) is configured to effect movement at a speed exceeding a speed of movement of the cutting assembly over a surface containing the hair intended to be cut.

9. A cutting assembly (400, 700) according to any of the preceding claims, wherein the movement mechanism (404, 704) comprises at least one of: a piezoelectric mechanism, a loudspeaker arrangement, a linear motor, and a capacitive micro-machined ultrasonic transducer, CMUT, device.

10. A cutting assembly (400, 700) according to any of the preceding claims, wherein the movement effected by the movement mechanism (404, 704) comprises a controlled vibration.

11. A cutting assembly (400, 700) according to claim 10, wherein the movement mechanism (404, 704) is configured to effect a controlled vibration having a vibrational frequency of between around 50 Hz and 15 kHz.

12. A cutting assembly (400, 700) according to any of the preceding claims, wherein the optical waveguide (402, 702) comprises an optical fiber.

13. A hair cutting device (800) for cutting hair on a body of a subject, the hair cutting device comprising:
a light source (104) for generating laser light at one or more specific wavelengths corresponding to wavelengths absorbed by one or more chromophores in hair; and
a cutting assembly (400, 700) having a cutting element coupled to the light source to receive laser light, the cutting assembly comprising a cutting assembly according to any of the preceding claims.

14. A hair cutting device (800) according to claim 13, further comprising:
a sensor (802) for measuring the speed of movement of the hair cutting device over the body of the subject.

15. A hair cutting device (800) according to claim 14, further comprising:
a processor (804) configured to vary the speed of movement effected by the movement mechanism based on the measured speed of movement of the hair cutting device.
